# EUROPEAN PATENT APPLICATION

(11) **EP 4 223 736 A1**
(43) Date of publication of application: **09.08.2023**
(21) Application number: 21875663.3
(22) Date of filing: 29.09.2021
(51) Int. Cl.: C07C 41/01, C07C 43/12, C07C 43/18, C07C 43/192, C07C 43/225, C07C 231/10, C07C 231/12, C07C 233/13, C07C 319/14, C07C 323/12

(54) **PRODUCTION METHOD FOR FLUORINATED ORGANIC COMPOUND**

(30) Priority: 30.09.2020 JP 2020165401; 12.08.2021 JP 2021131569
(71) Applicant: Daikin Industries, Ltd., Osaka-shi, Osaka 530-0001 (JP)
(72) Inventor: SHIRAI, Atsushi, Osaka-shi, Osaka 530-0001 (JP); NAMIKAWA, Takashi, Osaka-shi, Osaka 530-0001 (JP); ISHIHARA, Sumi, Osaka-shi, Osaka 530-0001 (JP); ADACHI, Kenji, Osaka-shi, Osaka 530-0001 (JP); KISHIKAWA, Yosuke, Osaka-shi, Osaka 530-0001 (JP); KUROKI, Yoshichika, Osaka-shi, Osaka 530-0001 (JP)
(74) Representative: Hoffmann Eitle
(86) International application number: PCT/JP2021/035763
(87) International publication number: WO 2022/071363

(57) **Abstract**

Provided is a novel production method for a fluorinated organic compound (1), the method comprising step A of fluorinating a hydrogen atom-containing organic compound (2) using IF₅ in a liquid composition comprising an organic solvent, the amount of the hydrogen atom-containing organic compound (2) being 1.8 mol or more per liter of the organic solvent.

## Description

### Technical Field

The present disclosure relates to a production method for a fluorinated organic compound.

### Background Art

Fluorine compounds are extremely important compounds as various chemical products, such as functional materials, pharmaceutical and agrochemical compounds, and electronic materials, and as intermediates thereof.

Fluorine, hydrogen fluoride, sulfur tetrafluoride, etc. have been conventionally used as fluorinating agents to obtain a target fluorine compound by fluorinating various organic compounds as a starting material. However, handling of these fluorinating agents requires special equipment and techniques.

A reaction for introducing a fluorine atom into an organic compound by using nucleophilic substitution with a fluoride ion has recently been developed, in addition to various fluorinating agents for use in the reaction.

For example, iodine pentafluoride (IF₅) is known as a powerful fluorinating agent with high oxidizability; however, it is a dangerous liquid fluorinating agent because it reacts with moisture in air and decomposes while generating HF. It has recently been reported that IF₅ having such features becomes a stable white solid (IF₅-pyridine-HF) in air when mixed with pyridine and HF, and is effective for fluorination of various sulfur compounds etc. (see Non-patent Literature (NPL) 1 and NPL 2).

### Citation List

### Non-patent Literature

NPL 1: S. Hara, M. Monoi, R. Umemura, C. Fuse, Tetrahedron, 2012, 68, 10145-10150
NPL 2: Journal of Fluorine Chemistry, Volume 167, 2014, pages 101-104

### Summary of Invention

### Technical Problem

An object of the present disclosure is to provide, for example, a novel production method for a fluorinated organic compound.

### Solution to Problem

The present disclosure encompasses the following embodiments.

### Item 1.

A production method for a fluorinated organic compound (1), comprising
step A of fluorinating a hydrogen atom-containing organic compound (2) using IF₅ in a liquid composition comprising an organic solvent, the amount of the hydrogen atom-containing organic compound (2) being 1.8 mol or more per liter of the organic solvent.

### Item 2.

The production method for a fluorinated organic compound according to Item 1, wherein the fluorination is performed using IF₅, an acid, and a base.

### Item 3.

The production method for a fluorinated organic compound according to Item 1 or 2, wherein the fluorination is performed using IF₅, HF, and an organic base.

### Item 4.

The production method for a fluorinated organic compound according to any one of Items 1 to 3, wherein the fluorination is performed using IF₅, HF, and an amine.

### Item 5.

The production method according to any one of Items 1 to 4, wherein the hydrogen atom-containing organic compound (2) is a compound having a partial structure: -C(=S)-Y-, wherein Y is O, S, or a single bond.

### Item 6.

The production method according to any one of Items 1 to 5, wherein the fluorinated organic compound (1) is a compound having at least one partial structure: -CF₂- formed in step A.

### Item 7.

The production method according to any one of Items 1 to 6, wherein the fluorinated organic compound (1) is a compound having at least one partial structure: -CF₃ formed in step A.

### Item 8

The production method according to any one of Items 1 to 7, wherein the concentration of the organic compound (2) is 2.5 mol or more per liter of the organic solvent.

### Item 9.

The production method according to any one of Items 1 to 8, wherein the organic solvent is an aprotic solvent.

### Item 10.

The production method according to any one of Items 1 to 9, wherein the reaction temperature of the fluorination step A is lower than 120°C.

### Item 11.

A composition comprising
(1) an organic solvent and
(2) IF₅ in an amount of 1.8 mol or more per liter of the organic solvent.

### Item 12.

A composition comprising:
(1) an organic solvent in an amount greater than 20.0 mass% based on the total amount of the composition taken as 100 mass%;
(2) IF₅ in an amount of 1.8 mol or more per liter of the organic solvent; and
(3) an acid or a base, or a combination thereof.

### Item 13.

The composition according to Item 11 or 12, wherein the organic solvent is an aliphatic hydrocarbon, an aromatic hydrocarbon, a halogenated hydrocarbon, an ether, a ketone, an ester, a nitrile, or an amide, or a combination of two or more thereof.

### Item 14.

The composition according to any one of Items 11 to 13, wherein the organic solvent is an aliphatic hydrocarbon, an aromatic hydrocarbon, a halogenated hydrocarbon, a nitrile, or a combination of two or more thereof.

### Item 15.

The composition according to any one of Items 11 to 14, wherein the acid is a Bronsted acid or a Lewis acid, or a combination thereof.

### Item 16.

The composition according to any one of Items 11 to 15, wherein the acid is a Bronsted acid.

### Item 17.

The composition according to any one of Items 11 to 16, wherein the acid is hydrogen fluoride.

### Item 18.

The composition according to any one of Items 11 to 17, wherein the base is an inorganic base or an organic base, or a combination thereof.

### Item 19.

The composition according to any one of Items 11 to 18, wherein the base is an organic base.

### Item 20.

The composition according to any one of Items 11 to 19, wherein the base is triethylamine or pyridine, or a combination thereof.

### Item 21.

The composition according to any one of Items 11 to 20, which is a liquid.

### Advantageous Effects of Invention

The present disclosure provides, for example, a novel production method for a fluorinated organic compound.

### Description of Embodiments

Although the details and form of the production method, composition, and the like according to the present disclosure are described below, it can be understood that various modifications in details and form may be made without departing from the spirit and scope of the claims.

Below, the production method for a fluorinated organic compound according to the present disclosure is described in detail.

### Terms

Unless otherwise specified, the symbols and abbreviations in the present specification are understood in the sense commonly used in the technical field to which the present disclosure pertains, according to the context of the present specification.

In the present specification, the terms "comprise" and "contain" are used with the intention of including the phrases "consisting essentially of" and "consisting of."

Unless otherwise specified, the steps, treatments, or operations described in the present specification can be performed at room temperature.

In the present specification, room temperature means a temperature in the range of 10 to 40°C, preferably 15 to 30°C.

### Production Method

The production method of the present disclosure is a method for producing a fluorinated organic compound (1), the method comprising
step A of fluorinating a hydrogen atom-containing organic compound (2) using IF₅ in a composition comprising an organic solvent, the amount of the hydrogen atom-containing organic compound (2) being 1.8 mol or more per liter of the organic solvent.

In the present disclosure, examples of the hydrogen atom-containing organic compound (2) as a reaction substrate include
(2-1) compounds having an OH group,
(2-2) ketone compounds (including diketones, β-ketocarboxylic acids, β-ketoesters, cyclic ketones, ketals, etc.), aldehyde compounds (including acetals), imine compounds, such as Schiff bases and hydrazones, or ester compounds,
(2-3) sulfide compounds,
(2-4) epoxy compounds,
(2-5) aromatic compounds (e.g., phenylhydrazine derivatives, phenol derivatives, 2-naphthol derivatives, and aniline derivatives),
(2-6) thiocarbonyl compounds,
(2-7) polyfluorination of the ethyl moiety of -COOR group-containing ethyl sulfide compounds, and
(2-8) unsaturated carbon compounds (e.g., olefin compounds).

The fluorination of organic compounds according to the present disclosure means substitution of a hydrogen atom with a fluorine atom, as well as substitution (replacement) of the following atom or groups with a fluorine atom as shown in the subsequent parentheses:
hydrogen atom (CH → CF),
carbonyl group (CO → CF₂),
thiocarbonyl group (CS → CF₂),
sulfide group (C-S- -> C-F),
hydrazino group (C-NHNH₂ → C-F; C=N-NH₂ → CF₂),
hydroxyl group (C-OH → C-F), and
epoxy group (C-O- → C-F).

Below, examples of fluorination in the production method according to the present disclosure are described. These examples also show examples of target products, i.e., fluorinated organic compounds, obtained by the production method of the present disclosure (fluorinated organic compounds (2)), as well as examples of substrates (organic compounds (1)).

### Fluorination Reaction 1: Fluorination of (2-1) compounds having an OH group

In this fluorination, for example, the following reactions are performed.

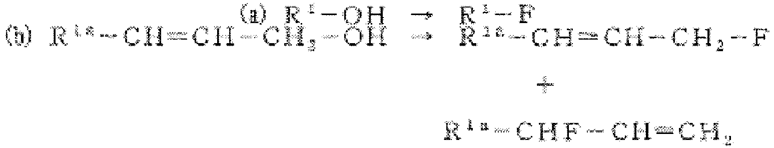

In the formulas,
R¹ represents an alkyl group optionally having at least one substituent, an aralkyl group optionally having at least one substituent, an alkenyl group optionally having at least one substituent, an acyl group, a cycloalkyl group optionally having at least one substituent, or a heterocycloalkyl group optionally having at least one substituent.
R^{1a} represents an alkyl group optionally having at least one substituent, an aryl group optionally having at least one substituent, an aralkyl group optionally having at least one substituent, an alkenyl group optionally having at least one substituent, an acyl group, a cycloalkyl group optionally having at least one substituent, or a heterocycloalkyl group optionally having at least one substituent.

In the present specification, "optionally having a substituent" includes both cases of having a substituent (i.e., substituted) and not having a substituent (i.e., unsubstituted). For example, an alkyl group optionally having a substituent includes an alkyl group (i.e., an unsubstituted alkyl group) and an alkyl group having a substituent (i.e., a substituted alkyl group).

Specific examples of compounds having an OH group include
aliphatic alcohols, such as methanol, ethanol, n-propanol, isopropanol, n-butanol, isobutanol, tert-butanol, pentanol, hexanol, octanol, decanol, palmityl alcohol, stearyl alcohol, and oleyl alcohol;
alicyclic alcohols, such as benzyl alcohol, a mono-, di- or trisaccharide having at least one non-protected hydroxyl group, cyclohexyl alcohol, and ascorbic acid; alcohol compounds, such as steroid alcohols (e.g., cholesterol, cholic acid, and cortisone);
aliphatic monocarboxylic acids, such as acetic acid, trifluoroacetic acid, propionic acid, acrylic acid, methacrylic acid, crotonic acid, butyric acid, valeric acid, isovaleric acid, pivalic acid, lauric acid, myristic acid, palmitic acid, stearic acid, oleic acid, linoleic acid, linolenic acid, and cinnamic acid;
polycarboxylic acids, such as oxalic acid, succinic acid, malonic acid, glutaric acid, adipic acid, maleic acid, fumaric acid, and citric acid;
aromatic carboxylic acids, such as benzoic acid, salicylic acid, (o-, m-, p-)phthalic acid, nalidixic acid, and nicotinic acid; vitamin compounds having carboxylic acid groups, such as pantothenic acid and biotin;
20 kinds of natural amino acids, such as glycine, alanine, phenylalanine, cysteine, aspartic acid, glutamic acid, threonine, histidine, lysine, methionine, and proline; and carboxylic acid compounds, such as hydroxycarboxylic acids (e.g., lactic acid, citric acid, malic acid, and tartaric acid).

### Fluorination Reaction 2: Fluorination of (2-2) ketone compounds (including diketones, β-ketocarboxylic acids, β-ketoesters, cyclic ketones, ketals, etc.), aldehyde compounds (including acetals), imine compounds, such as Schiff bases and hydrazones, or ester compounds

In this fluorination, for example, the following reactions are performed.

(a-1) R²-CH₂-C(=X)-R^{3a} → R²-CHF-C(=X)-R^{2a} → R²-CF₃-C(=X)-R^{3a}

(a-2) H-CH₂₋C(=X)-R^{2a} → H-CHF-C(=X)-R^{3a} → H-CF₂-C(=X)-R^{2a}

(a-3) R²-CH₂-C(=X)-H → R³-CHF-C(=X)-H → R²-CF₂-C(=X)-H

(b-I) R²-C(=X)-CH₂-C(=X)-R^{3a} → R²-C(=X)-CHF-C(=X)-R^{3a} → R²-C(=X)-CF₂-C(=X)-R^{2a}

(b~2) H-C(=X)-CH₂-C(=X)-R^{2a} → H-C(=X)-CHF-C(=X)-R^{2a} → H-C(=X)-CF₂-C(=X)-R^{2a}

(c) R³-C(=X)-R^{3a} → R³-CF₂-R^{2a} (R²)₂CH-COOR^{2b} → (R²)₂CF-COOR^{2b}

(d-1) R²-C(=N-NHR^{2c})-R^{2a} → R²-CF(-N=NR^{2c})-R^{2a} → R²-CF₂-R^{2a}

(d-2) HC(=N-NHR²)-R^{3a} → F₂C(-N=NR²)-R^{2a} → CF₃-R^{2a}

In the formulas,
X represents O or NR' (R' represents a hydrogen atom, an alkyl group optionally having at least one substituent, an aralkyl group optionally having at least one substituent, an aryl group optionally having at least one substituent, an alkenyl group optionally having at least one substituent, a cycloalkyl group optionally having at least one substituent, a heterocycloalkyl group optionally having at least one substituent, a heterocyclic group optionally having at least one substituent, an alkoxy group optionally having at least one substituent, an aryloxy group optionally having at least one substituent, an amino group, a monoalkylamino group optionally having at least one substituent, a dialkylamino group optionally having at least one substituent, an acyl group, or an acylamino group).
R², R^{2a}, and R^{2c} may be the same or different, and each represents a hydrogen atom, an alkyl group optionally having at least one substituent, an aralkyl group optionally having at least one substituent, an aryl group optionally having at least one substituent, an alkenyl group optionally having at least one substituent, a cycloalkyl group optionally having at least one substituent, a heterocycloalkyl group optionally having at least one substituent, a heterocyclic group optionally having at least one substituent, an alkoxy group optionally having at least one substituent, an aryloxy group optionally having at least one substituent, a monoalkylamino group optionally having at least one substituent, a dialkylamino group optionally having at least one substituent, an acyl group, or an acylamino group. Alternatively, R² and R^{2a} may be bonded to each other to form a ring structure.
R^{2b} represents an alkyl group optionally having at least one substituent, an aralkyl group optionally having at least one substituent, or an aryl group optionally having at least one substituent.

Examples of the ring structure include 4- to 7-membered aliphatic rings optionally having at least one substituent.

Examples of ketone compounds include acetone, methyl ethyl ketone, acetylacetone, acetoacetic acid, acetoacetate (e.g., methyl acetoacetate, ethyl acetoacetate), cyclohexanone, acetophenone, benzophenone, propiophenone, 4-piperidone, 1-oxo-1,2-dihydronaphthalene, benzylideneacetophenone (chalcone), and deoxybenzoin, and ketals thereof.

Examples of aldehyde compounds include acetaldehyde, propionaldehyde, butylaldehyde, isobutylaldehyde, valeraldehyde, isovaleraldehyde, acrylaldehyde, benzaldehyde, cinnamaldehyde, anisaldehyde, and nicotinaldehyde, and acetals thereof.

Examples of imine compounds, such as Schiff bases and hydrazones, include condensates of a ketone or an aldehyde with an appropriate primary amine or an appropriate hydrazine.

Examples of ester compounds include methyl isobutyrate and ethyl isobutyrate.

### Fluorination Reaction 3: Fluorination of (2-3) sulfide compounds (including dithioacetals and dithioketals)

In this fluorination, for example, reactions in which one or two hydrogen atoms of methylene adjacent to a sulfur atom are substituted with fluorine atom(s), or a sulfur atom is substituted with fluorine, are performed.

(a-1) R³-CH₂-S-R^{3a} → R³-CFH-S-R^{3a} → R³-CF₂-S-R^{3a}

(&-2) R³-CHR^{3b}-S-R^{3a} → R³-CFR^{3b}-S-R^{3a}

(b-1) R³-CO-CH₃-S-R^{3a} → R³-CO-CFH-S-R^{3a} → R³-CO-CF₂-S-R^{3a}

(b-2) R³-CO-CHR^{3b}-S-R^{3a} → R³-CO-CFR^{3b}-S-R^{3a}

(c) R^{3c}R^{3d}C=C(SR^{3a})₂, R^{3c}R^{3d}CH-CF₂-SR^{3a} → R^{3c}R^{3d}CH-CF₃

(d) R^{3c}R^{3d}C(SR^{3a'})(SR^{3a"}) → R^{3c}R^{3d}CF₃

(e) R³-C(SR^{3a})(SR^{3a'})(SR^{3a"}) → R³-CF₃

(f) R³-C(SR^{3a})(SR^{3a'})-S-R^{3e}-S-(SR^{3a'})-(SR^{3a})-R³ → R³-CF₃

In the formulas,
R^{3a}, R^{3a'}, and R^{3a"} may be the same or different, and each represents an alkyl group optionally having at least one substituent, an aralkyl group optionally having at least one substituent, an aryl group optionally having at least one substituent, an alkenyl group optionally having at least one substituent, a cycloalkyl group optionally having at least one substituent, a heterocycloalkyl group optionally having at least one substituent, or a heterocyclic group optionally having at least one substituent. Alternately, R^{3a} and R^{3a'} may be bonded to each other to form a 4- to 7-membered aliphatic ring optionally having at least one substituent.
R³ and R^{3b} may be the same or different, and each represents an alkyl group optionally having at least one substituent, an aralkyl group optionally having at least one substituent, an aryl group optionally having at least one substituent, an alkenyl group optionally having at least one substituent, a cycloalkyl group optionally having at least one substituent, a heterocycloalkyl group optionally having at least one substituent, a heterocyclic group optionally having at least one substituent, an alkoxy group optionally having at least one substituent, an aryloxy group optionally having at least one substituent, an amino group, a monoalkylamino group optionally having at least one substituent, a dialkylamino group optionally having at least one substituent, an acyl group, an acylamino group, a cyano group, an alkylsulfinyl group optionally having at least one substituent, an aralkylsulfinyl group optionally having at least one substituent, an arylsulfinyl group optionally having at least one substituent, a cycloalkylsulfinyl group optionally having at least one substituent, a heterocycloalkylsulfinyl group optionally having at least one substituent, a sulfinyl group to which a heterocyclic group optionally having at least one substituent is bonded, an alkylsulfonyl group optionally having at least one substituent, an aralkylsulfonyl group optionally having at least one substituent, an arylsulfonyl group optionally having at least one substituent, a cycloalkylsulfonyl group optionally having at least one substituent, a heterocycloalkylsulfonyl group optionally having at least one substituent, or a sulfonyl group to which a heterocyclic group optionally having at least one substituent is bonded. Alternately, R³ and R^{3b}, taken together with the carbon atom to which they are attached, optionally form a 4- to 8-membered ring via or not via a heteroatom (the ring is optionally substituted with at least one substituent selected from the group consisting of a halogen atom, an oxo group, an alkyl group optionally having at least one substituent, an aralkyl group optionally having at least one substituent, an aryl group optionally having at least one substituent, an alkenyl group optionally having at least one substituent, a cyano group, and an amino group.)
R^{3c} and R^{3d} may be the same or different, and each represents a hydrogen atom, an alkyl group optionally having at least one substituent, an aralkyl group optionally having at least one substituent, an aryl group optionally having at least one substituent, an alkenyl group optionally having at least one substituent, a cycloalkyl group optionally having at least one substituent, a heterocycloalkyl group optionally having at least one substituent, a heterocyclic group optionally having at least one substituent, an alkoxy group optionally having at least one substituent, an aryloxy group optionally having at least one substituent, a monoalkylamino group optionally having at least one substituent, a dialkylamino group optionally having at least one substituent, an acyl group, or an acylamino group. Alternately, R^{3c} and R^{3d}, taken together with the adjacent carbon atom, optionally form a saturated or unsaturated 4- to 7-membered aliphatic ring optionally having at least one substituent (this ring may be substituted with at least one member selected from the group consisting of a halogen atom, an oxo group, an alkyl group optionally having at least one substituent, an aralkyl group optionally having at least one substituent, an aryl group optionally having at least one substituent, an alkenyl group optionally having at least one substituent, a cyano group, and an amino group.)

Examples of sulfide compounds include methyl ethyl sulfide, methyl benzyl sulfide, 2-phenylthioacetate, 2-phenylthioacetophenone, 2-(methylthio)acetophenone, bis(methylthio)methylbenzene, 2-octyl-1,3-dithiane, 2-phenyl-2-trifluoromethyl-1,3-dithiolane, tris(ethylthio)hexane, and 4-tris(methylthio)toluene.

### Fluorination Reaction 4: Fluorination of (2-4) epoxy compounds

In this fluorination, for example, the following fluorine addition reaction is performed.

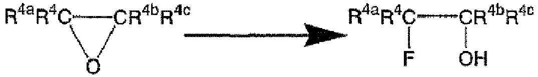

In the formula,
R⁴, R^{4a}, R^{4b}, and R^{4c} may be the same or different, and each represents a hydrogen atom, an alkyl group optionally having at least one substituent, an aralkyl group optionally having at least one substituent, an aryl group optionally having at least one substituent, an alkenyl group optionally having at least one substituent, a cycloalkyl group optionally having at least one substituent, a heterocycloalkyl group optionally having at least one substituent, or a heterocyclic group optionally having at least one substituent.

Examples of epoxy compounds include oxirane, 1,2-epoxyethylbenzene, 1-chloro-2,3-epoxypropane, and α,α'-epoxybibenzyl.

### Fluorination Reaction 5: Fluorination of (2-5) aromatic compounds

In this fluorination, for example, one or more fluorine substituents are introduced into the aromatic ring according to the following reactions. Fluorination of the aromatic ring in a phenol derivative or an aniline derivative can be performed by fluorinating it and then reducing it with a reducing agent, such as zinc dust, to obtain a target fluorinated compound.

### Fluorination Reaction 5-1: Fluorination of phenylhydrazine derivatives

A phenylhydrazine residue optionally having at least one substituent can be substituted with a fluorine atom.

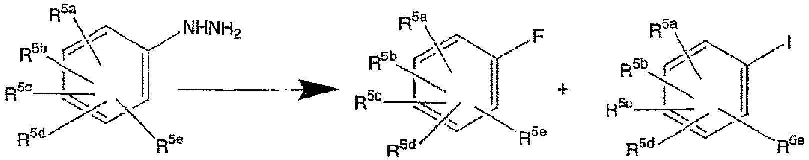

In the formula,
R^{5a}, R^{5b}, R^{5c}, R^{5d}, and R^{5e} may be the same or different, and each represents
a hydrogen atom, an alkyl group optionally having at least one substituent, an aralkyl group optionally having at least one substituent, an aryl group optionally having at least one substituent, an alkoxy group optionally having at least one substituent, a nitro group, a cyano group, a halogen atom, an acyl group, an arylcarbonyl group optionally having at least one substituent, an amino group, a monoalkylamino group optionally having at least one substituent, a dialkylamino group optionally having at least one substituent, an acylamino group optionally having at least one substituent, an arylcarbonylamino group optionally having at least one substituent, or an alkylthio group optionally having at least one substituent.

### Fluorination Reaction 5-2: Fluorination of phenol derivatives

Phenol derivatives form difluorinated quinonoid structures when reacted with IF₅ as shown below, and subsequent reduction of the resulting compounds produces phenol derivatives having fluorine introduced in the ortho- or para-position.

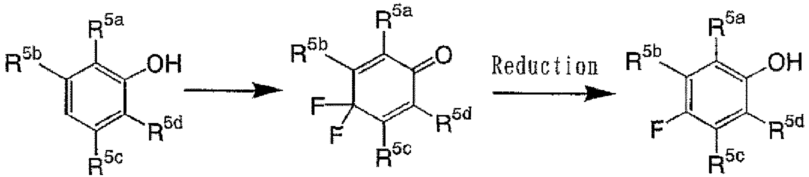

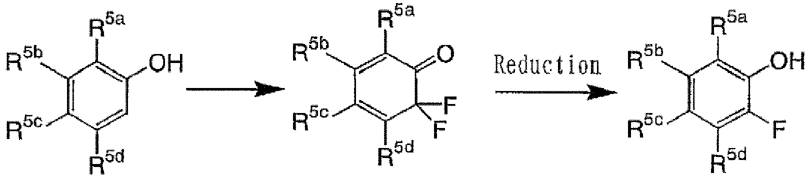

In the formulas,
R^{5a}, R^{5b}, R^{5c}, and R^{5d} may be the same or different, and each represents a hydrogen atom, an alkyl group optionally having at least one substituent, an aralkyl group optionally having at least one substituent, an aryl group optionally having at least one substituent, an alkoxy group optionally having at least one substituent, a nitro group, a cyano group, a halogen atom, an acyl group, an arylcarbonyl group optionally having at least one substituent, an amino group, a monoalkylamino group optionally having at least one substituent, dialkylamino group optionally having at least one substituent, an acylamino group optionally having at least one substituent, an arylcarbonylamino optionally having at least one substituent, or an alkylthio group optionally having at least one substituent.

When a starting material substituted at all of the ortho- and para-positions is used, a fluorine atom is introduced into the ortho-position or para-position, thereby forming a compound having a fluorinated quinonoid structure.

In the above example, phenol optionally having at least one substituent is used as a phenol derivative; however, it is also possible to use benzene-based aromatic compounds or condensed polycyclic hydrocarbons that have an electron-releasing group, such as a hydroxyl group or an alkoxy group, and that are optionally further substituted, to introduce a fluorine atom in a similar manner.

### Fluorination reaction 5-3: Fluorination of 2-naphthol derivatives

Mono- or di-fluorination at the 1-position of naphthol can be performed.

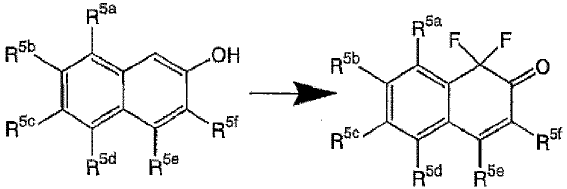

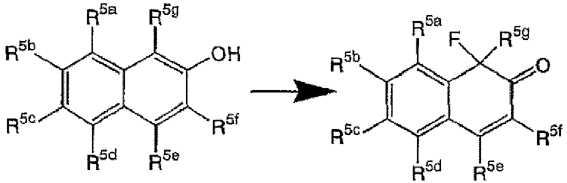

In the formulas,
R^{5a}, R^{5b}, R^{5c}, R^{5d}, R^{5e}, R^{5f}, and R^{5g} may be the same or different, and each represents a hydrogen atom, an alkyl group optionally having at least one substituent, an aralkyl group optionally having at least one substituent, an aryl group optionally having at least one substituent, an alkoxy group optionally having at least one substituent, a nitro group, a cyano group, a halogen atom, an acyl group, an arylcarbonyl group optionally having at least one substituent, an amino group, a monoalkylamino group, a dialkylamino group, an acylamino group, an arylcarbonylamino, or an alkylthio group optionally having at least one substituent.

### Fluorination Reaction 5-4: Fluorination of aniline derivatives

Like phenol derivatives, aniline derivatives also form difluorinated quinonoid structures when reacted with IF₅ as shown below, and subsequent reduction of the resulting compounds produces aniline derivatives having fluorine introduced in the ortho- or para-position.

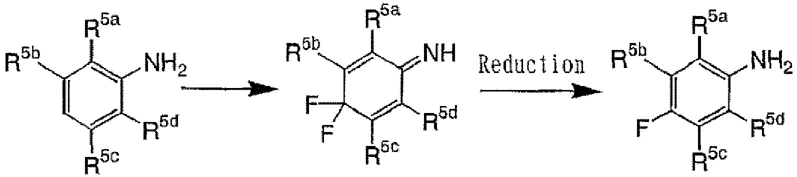

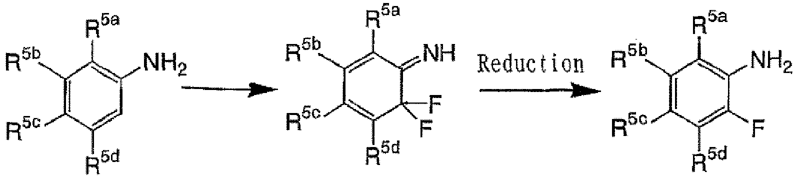

In the formulas,
R^{5a}, R^{5b}, R^{5c}, and R^{5d} may be the same or different, and each represents a hydrogen atom, an alkyl group optionally having at least one substituent, an aralkyl group optionally having at least one substituent, an aryl group optionally having at least one substituent, an alkoxy group optionally having at least one substituent, a nitro group, a cyano group, a halogen atom, an acyl group, an arylcarbonyl group optionally having at least one substituent, an amino group, a monoalkylamino group, a dialkylamino group, an acylamino group, an arylcarbonylamino, or an alkylthio group optionally having at least one substituent.

It is also possible to use an aniline derivative, such as aniline optionally having at least one substituent or naphthylamine optionally having at least one substituent to introduce a fluorine atom into the aromatic ring in a similar manner.

### Fluorination Reaction 6: (2-6) Fluorination of thiocarbonyl compounds (including thioketone, thioester, thiocarbonic ester, thioamide, dithiocarboxylate, and dithiocarbamate)

This reaction is for fluorination of a compound having the partial structure: -C(=S)-Y-,
wherein Y is O, S, or a single bond. For example, the following reactions can be performed.

(a) R⁶-C(=S)-R^{6a} → R⁶-CF₂-R^{6a}

(b) R⁶-C(=S)-SR^{6b} → R⁶-CF₂-SR^{6b} → R⁶-CF₃

In the formulas,
R⁶ and R^{6a} may be the same or different, and each represents a hydrogen atom, an alkyl group optionally having at least one substituent, an aralkyl group optionally having at least one substituent, an aryl group optionally having at least one substituent, an alkenyl group optionally having at least one substituent, a cycloalkyl group optionally having at least one substituent, a heterocycloalkyl group optionally having at least one substituent, a heterocyclic group optionally having at least one substituent, an alkoxy group optionally having at least one substituent, an aryloxy group optionally having at least one substituent, a monoalkylamino group optionally having at least one substituent, a dialkylamino group optionally having at least one substituent, an acyl group, or an acylamino group. Alternatively, R⁶ and R^{6a} may be bonded to each other to form a ring structure.
R^{6b} represents an alkyl group optionally having at least one substituent, an aralkyl group optionally having at least one substituent, an aryl group optionally having at least one substituent, an alkenyl group optionally having at least one substituent, a cycloalkyl group optionally having at least one substituent, a heterocycloalkyl group optionally having at least one substituent, or a heterocyclic group optionally having at least one substituent).

In the reaction of formula (b), when the amount of the fluorinating agent for use is reduced (e.g., 0.5 equivalents or less), the amount of the formation of R⁶-CF₂-SR^{6b} will be dominant.

Examples of thiocarbonyl compounds include O-(4-isopropylphenyl)S-methyl dithiocarbonate, O-(4-bromophenyl)S-methyl dithiocarbonate, ethyl 4-(((methylthio)carbonothioyl)oxy) benzoate, O-decyl S-methyl dithiocarbonate, O-(3-phenylpropyl)S-methyl dithiocarbonate, O-methyl cyclohexanecarbothioate, O-propyl 1-piperidinecarbothioate, methyl dithiobenzoate, thiobenzophenone, O-phenyl thiobenzoate, N,N-dimethylphenylthioamide, ethyl 3-quinolinedithiocarboxylate, trifluoromethanecarbothioylnaphthalene, N-methyl-N-phenyltrifluoromethanethioamide, N-benzyl-N-phenylheptafluoropropanethioamide, O-(4'-pentyl-[1,1'-bi(cyclohexane)]-4-yl)S-methyl dithiocarbonate, 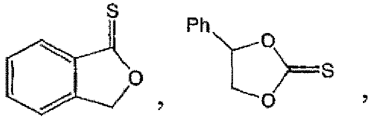 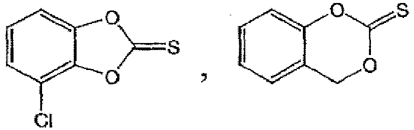 and the like.

### Fluorination Reaction 7: (2-7) Polyfluorination of the ethyl moiety of -COOR group-containing ethyl sulfide compounds

In this fluorination, the ethyl moiety adjacent to an S atom is polyfluorinated.

R⁷-S-CH(COOR^{7a})-CH₃ → R⁷-S-CHF-CF₂-COOR^{7a}

In the formula,
R⁷ represents an aryl group optionally having at least one substituent or an aromatic heterocyclic group optionally having at least one substituent.
R^{7a} represents a hydrogen atom, an alkyl group optionally having at least one substituent, an aralkyl group optionally having at least one substituent, an aryl group optionally having at least one substituent, an alkenyl group optionally having at least one substituent, a cycloalkyl group optionally having at least one substituent, a heterocycloalkyl group optionally having at least one substituent, a heterocyclic group optionally having at least one substituent, an alkoxy group optionally having at least one substituent, an aryloxy group optionally having at least one substituent, an amino group, a monoalkylamino group optionally having at least one substituent, a dialkylamino group optionally having at least one substituent, an acyl group, an acylamino group, a cyano group, an alkylsulfinyl group optionally having at least one substituent, an aralkylsulfinyl group optionally having at least one substituent, an arylsulfinyl group optionally having at least one substituent, a cycloalkylsulfinyl group optionally having at least one substituent, a heterocycloalkylsulfinyl group optionally having at least one substituent, a sulfinyl group to which a heterocyclic group optionally having at least one substituent is bonded, an alkylsulfonyl group optionally having at least one substituent, an aralkylsulfonyl group optionally having at least one substituent, an arylsulfonyl group optionally having at least one substituent, a cycloalkylsulfonyl group optionally having at least one substituent, a heterocycloalkylsulfonyl group optionally having at least one substituent, or a sulfonyl group to which a heterocyclic group optionally having at least one substituent is bonded.

Examples of -COOR group-containing ethyl sulfide compounds include 2-((4-chlorophenyl)thio)propanoic acid ethyl ester.

### Fluorination Reaction 8: Fluorination of (2-8) unsaturated carbon compounds

In this fluorination, fluorine or iodine is added to a carbon-carbon double bond or carbon-carbon triple bond.

(a) R^{8a}R^{8a'}C=CR^{8b}R^{8b'} → FR^{8a}C-CR^{8b}I

(b) R^{8a}C≡CR^{8b} → FR^{8a}C=CR^{8b}I

In the formulas,
R^{8a}, R^{8a'}, R^{8b}, and R^{8b'} may be the same or different, and each represents a hydrogen atom, an alkyl group optionally having at least one substituent, an aryl group optionally having at least one substituent, an aralkyl group optionally having at least one substituent, an alkenyl group optionally having at least one substituent, an acyl group, a cycloalkyl group optionally having at least one substituent, a heterocycloalkyl group optionally having at least one substituent, an ester group, or a halogen atom. Alternatively, at least two of the R^{8a}, R^{8a'}, R^{8b}, and R^{8b'} may be bonded to each other to form a ring structure.

Examples of the ring structure include 4- to 12-membered aliphatic rings optionally having at least one substituent.

Examples of unsaturated carbon compounds include C₂-C₂₀ unsaturated carbon compounds, such as decene, cyclodecene, and dodecyne.

In the present specification, examples of alkyl groups include linear or branched C₁-C₁₈ alkyl groups, such as methyl, ethyl, n-propyl, isopropyl, n-butyl, isobutyl, sec-butyl, tert-butyl, pentyl, hexyl, heptyl, octyl, nonyl, decyl, undecyl, dodecyl, tridecyl, tetradecyl, pentadecyl, hexadecyl, heptadecyl, and octadecyl. Preferred are linear or branched C₁-C₆ alkyl groups, such as methyl, ethyl, n-propyl, isopropyl, n-butyl, isobutyl, sec-butyl, tert-butyl, pentyl, and hexyl.

In the present specification, examples of alkoxy groups include linear or branched C₁-C₆ alkoxy groups, such as methoxy, ethoxy, n-propoxy, isopropoxy, n-butoxy, isobutoxy, sec-butoxy, tert-butoxy, pentyloxy, and hexyloxy.

In the present specification, examples of alkenyl groups include C₂-C₆ alkenyl groups, such as a vinyl group, an allyl group, and a 3-butenyl group.

In the present specification, examples of halogen atoms include a fluorine atom, a chlorine atom, a bromine atom, and an iodine atom.

In the present specification, examples of aryl groups include a phenyl group and a naphthyl group.

In the present specification, examples of aryloxy groups include a phenoxy group and a naphthyloxy group.

In the present specification, examples of aralkyl groups include C₇-C₁₀ aralkyl groups, such as 2-phenylethyl, benzyl, 1-phenylethyl, 3-phenylpropyl, and 4-phenylbutyl.

In the present specification, examples of cycloalkyl groups include C₃-C₈ cycloalkyl groups, such as cyclopropyl, cyclobutyl, cyclopentyl, cyclohexyl, cycloheptyl, and cyclooctyl. Preferred are C₃-C₇ cycloalkyl groups.

In the present specification, examples of heterocycloalkyl groups include groups in which one or more carbon atoms that constitute the ring structure of the cycloalkyl groups described above are substituted with nitrogen, oxygen, sulfur, etc.

In the present specification, examples of monoalkylamino groups include amino groups mono-substituted with the C₁-C₆ alkyl groups described above.

In the present specification, examples of dialkylamino groups include amino groups di-substituted with the C₁-C₆ alkyl groups described above, such as dimethylamino, diethylamino, din-propylamino, diisopropylamino, dibutylamino, dipentylamino, and dihexylamino.

In the present specification, examples of acylamino groups include formylamino, benzoylamino, acetylamino, propionylamino, n-butyrylamino, and like C₁-C₈ acylamino groups (e.g., formylamino, alkanoylamino, and arylcarbonylamino groups).

Examples of alkylthio groups include -S-(C₁-C₆ alkyl groups) (wherein C₁-C₆ alkyl groups are as defined above.)

In the present specification, examples of heterocyclic groups include 5- to 10-membered monocyclic or bicyclic heterocyclic groups having at least one heteroatom selected from nitrogen, oxygen, and sulfur as a ring-constituting atom, such as piperidyl, furyl, thienyl, imidazolyl, oxazolyl, thiazolyl, pyrrolyl, pyrrolidinyl, triazolyl, benzothiazolyl, benzimidazolyl, oxadiazolyl, thiadiazolyl, indolyl, pyrazolyl, pyridazinyl, cinnolinyl, quinolyl, isoquinolyl, quinoxalinyl, pyrazinyl, pyridyl, benzofuryl, benzothienyl, and tetrazolyl.

In the present specification, of the heterocyclic groups, examples of aromatic heterocyclic groups include 5- to 10-membered monocyclic or bicyclic heteroaryl groups having at least one heteroatom selected from nitrogen, oxygen, and sulfur as a ring-constituting atom, such as furyl, thienyl, imidazolyl, oxazolyl, thiazolyl, pyrrolyl, triazolyl, benzothiazolyl, benzimidazolyl, oxadiazolyl, thiadiazolyl, indolyl, pyrazolyl, pyridazinyl, cinnolinyl, quinolinyl, isoquinolinyl, quinoxalinyl, pyrazinyl, pyridyl, benzofuryl, benzothienyl, and tetrazolyl.

In the present specification, examples of acyl groups include a formyl group; linear or branched C₂-C₆ alkanoyl groups, such as acetyl, propionyl, n-butyryl, isobutyryl, valeryl, isovaleryl, and pivaloyl; and C₇-C₁₅ arylcarbonyl groups, such as benzoyl.

Examples of acyl groups include substituted acetyl groups, such as chloroacetyl, bromoacetyl, dichloroacetyl, and trifluoroacetyl;
alkoxy-substituted acetyl groups, such as methoxyacetyl and ethoxyacetyl, and alkylthio-substituted acetyl groups, such as methylthioacetyl; and
substituted benzoyl groups, such as phenoxyacetyl, phenylthioacetyl, 2-chlorobenzoyl, 3-chlorobenzoyl, 4-chlorobenzoyl, 4-methylbenzoyl, 4-t-butylbenzoyl, 4-methoxybenzoyl, 4-cyanobenzoyl, and 4-nitrobenzoyl.

In the present specification, examples of alkanoyl groups include linear or branched C₂-C₆ alkanoyl groups, such as acetyl, propionyl, n-butyryl, isobutyryl, valeryl, isovaleryl, and pivaloyl.

In the present specification, examples of the alkyl group, aralkyl group, aryl group, cycloalkyl group, heterocycloalkyl group, and heterocyclic group in an alkylsulfinyl group, aralkylsulfinyl group, arylsulfinyl group, cycloalkylsulfinyl group, heterocycloalkylsulfinyl group, and sulfinyl group to which a heterocyclic group is bonded include those described above.

In the present specification, examples of the alkyl group, aralkyl group, aryl group, cycloalkyl group, heterocycloalkyl group, and heterocyclic group in an alkylsulfonyl group, aralkylsulfonyl group, arylsulfonyl group, cycloalkylsulfonyl group, heterocycloalkylsulfonyl group, and sulfonyl group to which a heterocyclic group is bonded include those described above.

In the present specification, examples of the aryl group in an arylcarbonyl group include those described above.

In the present specification, examples of the arylcarbonyl group in an arylcarbonylamino group include those described above.

In the present specification, examples of esters include an acyl-O-group and an alkoxy-CO-group. Examples of the acyl and alkoxy used here include the acyl groups and alkoxy groups described above.

The number of substituents in an alkyl group having at least one substituent, an alkoxy group having at least one substituent, or an alkenyl group having at least one substituent may be 1 to 5, and preferably 1 to 3. Examples of substituents include a halogen atom, a C₁-C₆ alkoxy group, a C₁-C₆ alkylthio group, a cyano group, a nitro group, an amino group, and a hydroxyl group. Examples of an alkyl group having a halogen atom include an alkyl group in which a part or all of the hydrogen atoms are substituted with fluorine.

The number of substituents in an aralkyl group having at least one substituent, an aryl group having at least one substituent, an aryloxy group having at least one substituent, a cycloalkyl group having at least one substituent, a heterocycloalkyl group having at least one substituent, a heterocyclic group having at least one substituent, a monoalkylamino group having at least one substituent, a dialkylamino group having at least one substituent, an acylamino group, an alkylsulfinyl group having at least one substituent, an aralkylsulfinyl group having at least one substituent, an arylsulfinyl group having at least one substituent, a cycloalkylsulfinyl group having at least one substituent, a heterocycloalkylsulfinyl group having at least one substituent, a sulfinyl group to which a heterocyclic group having at least one substituent is bonded, an alkylsulfonyl group having at least one substituent, an aralkylsulfonyl group having at least one substituent, an arylsulfonyl group having at least one substituent, a cycloalkylsulfonyl group having at least one substituent, a heterocycloalkylsulfonyl group having at least one substituent, a sulfonyl group to which a heterocyclic group having at least one substituent is bonded, an arylcarbonyl group having at least one substituent, an acylamino group having at least one substituent, and an arylcarbonylamino group having at least one substituent may be, for example, 1 to 5, and preferably 1 to 3. Examples of substituents include a C₁-C₆ alkyl group, a halogen atom, a C₁-C₆ alkoxy group, a C₁-C₆ alkylthio group, a cyano group, a nitro group, an amino group, and a hydroxyl group.

The number of substituents in 4- to 7-membered aliphatic rings having at least one substituent may be 1 to 5, and preferably 1 to 3. Examples of substituents include a C₁-C₆ alkyl group, a halogen atom, a C₁-C₆ alkoxy group, a C₁-C₆ alkylthio group, a cyano group, a nitro group, an amino group, a hydroxyl group, and a carboxy ester.

Additionally, the following formula: 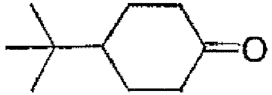 is also encompassed by 4- to 7-membered aliphatic rings having at least one substituent.

The lower limit of the amount of the hydrogen atom-containing organic compound (2) per liter of the organic solvent in step A is
1.8 mol or more,
preferably 2.0 mol or more,
more preferably 2.5 mol or more, and
still more preferably 2.8 mol or more.

An amount of the hydrogen atom-containing organic compound (2) in step A of less than 1.8 mol per liter of the organic solvent results in a reduction in the yield of the target fluorinated organic compound (1). IF₅ for use in the present disclosure is a strong oxidant. Thus, in view of common technical knowledge, an increase in the amount of the hydrogen atom-containing organic compound (2) is expected to also produce a greater amount of decomposed products, and the selectivity is assumed to decrease; thus, the yield is also expected to decrease. Nevertheless, in the present disclosure, the target product can be obtained in a high yield.

The upper limit of the amount of the hydrogen atom-containing organic compound (2) in step A is not limited, and may be, for example, 20 mol or less, 15 mol or less, or 10 mol or less, per liter of the organic solvent, from the standpoint of yield of the target fluorinated organic compound (1) and from an economic standpoint.

The lower limit of the amount of IF₅ per liter of the organic solvent in step A may be specifically, for example, preferably 1.8 mol or more,
more preferably 2.0 mol or more,
still more preferably 2.2 mol or more,
even more preferably 2.5 mol or more,
particularly preferably 2.7 mol or more,
more particularly preferably 3.0 mol or more, and
most preferably 3.5 mol or more, from the standpoint of yield of the target fluorinated organic compound (1).

When the amount of IF₅ in step A is 1.8 mol or more per liter of the organic solvent, the yield of the target fluorinated organic compound (1) can be easily further improved. IF₅ for use in the present disclosure is a strong oxidant. Thus, in view of common technical knowledge, an increase in the amount of IF₅ is expected to also produce a greater amount of decomposed products, and the selectivity is assumed to decrease; thus, the yield is also expected to decrease. Nevertheless, in the present disclosure, the target product can be easily obtained in a higher yield.

The upper limit of the amount of IF₅ in step A is not limited. Specifically, the amount per liter of the organic solvent may be, for example,
100 mol,
70 mol or less,
50 mol or less,
30 mol or less,
20 mol or less,
15 mol or less, or
10 mol or less, from the standpoint of yield of the target fluorinated organic compound (1) and from an economic standpoint.

The concentration of IF₅ in step A is not limited and is preferably approximately equal to the amount of the hydrogen atom-containing organic compound (2) (specifically, the molar ratio relative to the hydrogen atom-containing organic compound (2) is preferably in the range of 0.1 to 10.0, more preferably 0.3 to 5.0, and still more preferably 0.5 to 3.0), from the standpoint of yield of the target fluorinated organic compound (1).

The fluorination described above is performed using IF₅. From the standpoint of, for example, yield of the target fluorinated organic compound (1), the fluorination is preferably performed using IF₅, an acid, and a base.

The acid is preferably a Bronsted acid or a Lewis acid, or a combination thereof, from the standpoint of, for example, yield of the target fluorinated organic compound (1).

Specific examples of the acid include
hydrogen halides, hydrohalic acids, hypohalous acids, halous acids, halogen acids, and perhalogen acids, such as sulfuric acid, nitric acid, phosphoric acid, polyphosphoric acid, hydrogen fluoride (HF), hydrofluoric acid, hydrochloric acid, hydrogen bromide, hydrogen iodide, hypochlorous acid, chlorous acid, chloric acid, perchloric acid, perbromic acid, and periodic acid; sulfonic acids, such as fluorosulfonic acid, chlorosulfonic acid, methanesulfonic acid, ethanesulfonic acid,
trifluoromethanesulfonic acid, difluoromethanesulfonic acid, trichloromethanesulfonic acid, perfluorobutanesulfonic acid, perfluorooctanesulfonic acid, benzenesulfonic acid, toluenesulfonic acid, and nitrobenzenesulfonic acid, or polymers carrying sulfonic acids, such as polystyrenesulfonic acid and fluorinated sulfonic acid resin (e.g., Nafion-H);
mono- or polycarboxylic acids, such as formic acid, acetic acid, propionic acid, chloroacetic acid, bromacetic acid, dichloroacetic acid, trichloroacetic acid, trifluoroacetic acid, glycolic acid, lactic acid, benzoic acid, oxalic acid, and succinic acid;
Lewis acids and their ether complexes, such as SO₃, BF₃, BCl₃, B(OCH₃)₃, AlCl₃, AlBr₃, SbF₃, SbCl₃, SbF₅, PF₃, PF₅, AsF₃, AsCl₃, AsF₅, TiCl₄, NbF₅, and TaF₅;
acids formed of Lewis acids and hydrogen halides, and their ether complexes, such as HBF₄, HPF₆, HAsF₆, HSbF₆, and HSbCl₆; and mixtures of two or more thereof.

These acids may be supported by various carriers.

Examples of the carriers include SiO₂, methylated SiO₂, Al₂O₃, Al₂O₃-WB, MoO₃, ThO₂, ZrO₂, TiO₂, Cr₂O₃, SiO₂-Al₂O₃, SiO₂₋TiO₂, SiO₂-ZrO₂, TiO₂-ZrO₂, Al₂O₃-B₂O₃, SiO₂-WO₃, SiO₂-NH₄F, HSO₃Cl-Al₂O₃, HF-NH₄-Y, HF-Al₂O₃, NH₄F-SiO₂-Al₂O₃, AlF₃-Al₂O₃, Ru-F-Al₂O₃, F-Al₂O₃, KF-Al₂O₃, AlPO₄, AlF₃, bauxite, kaolin, activated carbon, graphite, Pt-graphite, ion-exchange resins, metal sulfates, chlorides, metals (e.g., Al), alloys (e.g., Al-Mg, Ni-Mo), and polymers (e.g., polystyrene).

Preferably, the acid may be, for example, HF from the standpoint of, for example, yield of the target fluorinated organic compound (1). Preferably, the base may be, for example, an organic base from the standpoint of, for example, yield of the target fluorinated organic compound (1), as described below in detail. Thus, preferably, the fluorination described above can be performed using, for example, IF₅, HF, and an organic base.

The amount of the acid per mole of IF₅ can be in the range of
preferably 0.5 to 20 mol,
more preferably 0.8 to 10 mol, and
still more preferably 0.9 to 5 mol, from the standpoint of, for example, yield of the target fluorinated organic compound (1).

Examples of the base include
alkali metal hydroxides or alkaline earth metal hydroxides, such as sodium hydroxide, potassium hydroxide, lithium hydroxide, rubidium hydroxide, cesium hydroxide, magnesium hydroxide, calcium hydroxide, and barium hydroxide;
alkali metal alkoxides, such as sodium methoxide, sodium ethoxide, sodium butoxide, potassium methoxide, potassium ethoxide, potassium butoxide, lithium methoxide, and lithium ethoxide; alkali metal hydrides or alkaline earth metal hydrides, such as sodium hydride, potassium hydride, lithium hydride, and calcium hydride;
alkali metals, such as sodium, potassium, and lithium;
alkaline earth metal oxides, such as magnesium oxide and calcium oxide;
ammonia and ammonium hydroxide salts, such as ammonium hydroxide, tetramethylammonium hydroxide, tetraethylammonium hydroxide, tetrabutylammonium hydroxide, octyltriethylammonium hydroxide, and benzyltrimethylammonium hydroxide, or polymers carrying ammonium hydroxide salts (e.g., Amberlite resin);
aliphatic amines (primary amines, secondary amines, tertiary amines), alicyclic amines (secondary amines, tertiary amines), aromatic amines (primary amines, secondary amines, tertiary amines), and heterocyclic amines; and
polymers carrying amine compounds, such as polyallylamine and polyvinylpyridine.

In the present specification, examples of aliphatic primary amines include methylamine, ethylamine, propylamine, butylamine, pentylamine, hexylamine, cyclohexylamine, and ethylenediamine.

In the present specification, examples of aliphatic secondary amines include dimethylamine, diethylamine, dipropylamine, dibutylamine, dipentylamine, dihexylamine, and dicyclohexylamine.

In the present specification, examples of aliphatic tertiary amines include trimethylamine, triethylamine, diisopropylethylamine, and N,N,N',N'-tetramethylethylenediamine.

In the present specification, examples of alicyclic secondary amines include piperidine, piperazine, pyrrolidine, and morpholine.

In the present specification, examples of alicyclic tertiary amines include N-methylpiperazine, N-methylpyrrolidine, 5-diazabicyclo[4.3.0]non-5-ene, and 1,4-diazabicyclo[2.2.2]octane.

In the present specification, examples of aromatic amines include aniline, methylaniline, dimethylaniline, N,N-dimethylaniline, haloaniline, and nitroaniline. In the present specification, examples of heterocyclic amines include pyridine, pyrimidine, piperazine, quinoline, and imidazole.

The base can be preferably, for example, an organic base, and more preferably, for example, an amine, from the standpoint of, for example, yield of the target fluorinated organic compound (1). That is, the fluorination described above is further preferably performed using IF₅, HF, and an amine. Examples of this amine include aliphatic amines, alicyclic amines, aromatic amines, and heterocyclic amines, as well as polymers carrying amine compounds.

The amount of the base per mole of IF₅ is
preferably in the range of 0.5 to 20 mol,
more preferably 0.8 to 10 mol,
still more preferably 0.9 to 5 mol, from the standpoint of, for example, yield of the target fluorinated organic compound (1).

The acid and the base may form salts.

Examples of the salts include
metal salts or ammonium salts of sulfuric acid or sulfonic acid, such as sodium sulfate, sodium hydrogensulfate, potassium sulfate, potassium hydrogensulfate, lithium sulfate, cesium sulfate, calcium sulfate, magnesium sulfate, ammonium sulfate, triethylammonium sulfate, pyridinium sulfate, trimethylpyridinium sulfate, polyallyammonium sulfate, polyvinylpyridinium sulfate, sodium methanesulfonate, ammonium methanesulfonate, tetramethylammonium methanesulfonate, potassium ethanesulfonate, lithium butanesulfonate, sodium benzenesulfonate, sodium toluenesulfonate, sodium trifluoromethanesulfonate, and sodium polystyrenesulfonate;
metal salts or ammonium salts of carboxylic acids, such as sodium formate, ammonium formate, sodium acetate, potassium acetate, lithium acetate, magnesium acetate, calcium acetate, ammonium acetate, methylammonium acetate, diethylammonium acetate, triethylammonium acetate, tetraethylammonium acetate, pyridinium acetate, sodium propionate, potassium propionate, sodium butyrate, polyallylammonium acetate, polyvinylpyridinium acetate, sodium isobutyrate, sodium valerianate, sodium nonanoate, sodium chloroacetate, sodium bromoacetate, sodium trichloroacetate, sodium trifluoroacetate, sodium glycolate, sodium lactate, sodium benzoate, sodium oxalate, sodium succinate, and sodium polyacrylate;
metal salts, such as LiBr, LiI, NaBr, NaI, KBr, KI, RbBr, RbI, CsBr, CsI, BeBr₂, BeI₂, MgBr₂, MgI₂, CaBr₂, CaI₂, SrBr₂, SrI₂, BaBr₂, BaI₂, ZnBr₂, ZnI₂, CuBr₂, CuI₂, CuBr, CuI, AgBr, AgI, AuBr, AuI, NiBr₂, NiI₂, PdBr₂, PdI₂, PtBr₂, PtI₂, CoBr₂, CoI₂, FeBr₂, FeBr₃, FeI₂, FeI₃, MnBr₂, MnI₂, CrBr₂, CrI₂, PbBr₂, PbI₂, SnBr₂, SnI₂, SnBr₄, and SnI₄;
pyridinium salts or ammonium salts, such as NH₄Br, NH₄I, MeNH₃Br, MeNH₃I, Me₄NBr, Me₄NI, Et₄NBr, Et₄NI, Bu₄NBr, Bu₄NI, PhMe₃NBr, PhMe₃NI, PhCH₂NMe₃I, pyridinium bromide, pyridinium iodide, chloropyridinium iodide, methylpyridinium iodide, cyanopyridinium iodide, bipyridinium iodide, quinolium iodide, isoquinolium iodide, N-methylpyridinium bromide, N-methylpyridinium iodide, and N-methylquinolium iodide;
phosphonium salts, such as Me₄PBr, Me₄PI, Et₄PI, Pr₄I, Bu₄PBr, Bu₄PI, Ph₄PBr, and Ph₄PI (wherein Me represents a methyl group, Et represents an ethyl group, Pr represents an n-propyl group, and Bu represents an n-butyl group);
metal salts or amine salts of hydrogen halides, hypohalous acids, halous acids, halogen acids, or perhalogen acids, such as sodium fluoride, potassium fluoride, cesium fluoride, ammonium fluoride, tetraethylammonium fluoride, tetrabutylammonium fluoride, polyallylammonium fluoride, sodium chloride, ammonium chloride, sodium hypochlorite, sodium chlorite, sodium chlorate, sodium perchlorate, sodium perbromate, and sodium periodate;
carbonates, such as sodium carbonate, potassium carbonate, lithium carbonate, sodium hydrogencarbonate, potassium hydrogencarbonate, lithium hydrogencarbonate, calcium carbonate, and magnesium carbonate;
metal salts or amine salts of phosphoric acids, such as sodium phosphate, potassium phosphate, sodium hydrogenphosphate, sodium dihydrogenphosphate, ammonium phosphate, and pyridinium phosphate;
metal salts or amine salts of nitric acid, such as sodium nitrate, potassium nitrate, ammonium nitrate, and pyridinium nitrate;
metal salts or amine salts formed of Lewis acids and hydrogen halides, such as NaBF₄, KBF₄, LiBF₄, NaSbF₆, NaAsF₆, NaPF₆, NH₄BF₄, NH₄SbF₆, and NH₄PF₆;
phosphonium salts, such as tetramethylphosphonium fluoride, tetramethylphosphonium acetate, and tetraphenylphosphonium fluoride; and
room-temperature molten salts having fluoride anions or HF, such as (C₂H₅)₄NF, 1-ethyl-3-methylimidazolium fluoride, (C₂H₅)₃N-(HF)ₙ, (C₂H₅)₄NF-(HF)ₙ, (n-C₄H₉)₃N-(HF)ₙ, (n-C₄H₉)₄NF-(HF)ₙ, BF₃•Et₂O-(HF)ₙ (wherein n is 1 to 20, and Et represents an ethyl group).

These salts may be used alone or as a mixture or a combination of two or more.

In the production method of the present disclosure, IF₅ may form a complex with the acid and/or the base.

If desired, additives, such as halogens, interhalogen compounds, and polyhalides, may be used with IF₅.

Examples of the halogens include iodine, bromine, and chlorine. Of these, iodine or bromine is preferred, and iodine is more preferred.

The "interhalogen compounds" means interhalogen compounds other than IF₅. Examples include ClF, BrF, ICl, IBr, I₂Cl₆, and ICl₃.

Examples of the polyhalides include LiCl₄I, NaCl₄I, KCl₄I, CsCl₄I, RbCl₄I, Me₄NCl₄I, Et₄NCl₄I, Pr₄NCl₄I, Bu₄NCl₄I, PhNMe₃Cl₄I, PhCH₂NMe₃Cl₄I, Me₃SCl₄I, Cl₈IP, KCl₃I₂, Me₄NCl₃I₂, 2,2'-bipyridinium µ-chlorodichlorodiiodate, 2,2'-biquinolinium µ-chlorodichlorodiiodate, KCl₂I, Me₄NCl₂I, Me₄NClI₂, Et₄NCl₃, Ph₄AsCl₃, KClF₂, Me₄NClF₄, CsClF₄, CsCl₃FI, KBrClI, NH₄BrClI, Me₄NBrClI, Me₄NBrCl₂, Bu₄NBrCl₂, Me₄NBrCl₂I₂, CsBrFI, NaBrF₂, KBrF₂, CsBrF₄, Me₄NBrF₄, CsBrF₆, Me₄NBrF₆, Et₄NBr₆Cl, CsBr₃, Me₄NBr₃, Et₄Br₃, Bu₄NBr₃, PhCH₂NMe₃Br₃, pyridinium tribromide, Br₇P, CsBrI₂, Me₄NBrI₂, Me₄NBrI₄, Me₄NBrI₆, KBr₂Cl, Me₄NBr₂Cl, Bu₄NBr₂Cl, KBr₂I, Me₄NBr₂I, Bu₄NBr₂I, 2,2'-bipyridinium p-bromodibromodiiodate, NaF₂I, KF₂I, CsF₄I, CsF₆I, CsF₈I, KI₃, CsI₃, Me₄NI₃, Et₄NI₃, Pr₄NI₃, Bu₄NI₃, pyridinium triiodide, Me₄NI₅, Et₄NI₇, Me₄NI₉, Me₄PBr₃, Me₄PI₃, Me₄PIBr₂, Me₄PICl₂, Et₄PI₃, Bu₄PI₃, Ph₄PI₃, Ph₄PBr₃, and Ph₄PIBr₂ (wherein Me represents a methyl group, Et represents an ethyl group, Pr represents an n-propyl group, Bu represents an n-butyl group, and Ph represents a phenyl group).

The additives may be used alone or as a mixture or a combination of two or more.

The hydrogen atom-containing organic compound (2) is preferably a compound having the partial structure: -C(=S)-Y-, wherein Y is O, S, or a single bond, and fluorination reaction 6 described above is preferred, from the standpoint of, for example, yield of the target fluorinated organic compound (1).

The fluorinated organic compound (1) is preferably a compound having at least one partial structure: -CF₂- formed in step A described above, from the standpoint of, for example, yield of the target fluorinated organic compound (1).

The fluorinated organic compound (1) is also preferably a compound having at least one partial structure: -CF₃ formed in step A described above, from the standpoint of, for example, yield of the target fluorinated organic compound (1).

The organic solvent is preferably an aprotic solvent from the standpoint of, for example, yield of the target fluorinated organic compound (1).

From the standpoint of, for example, yield of the target fluorinated organic compound (1), examples of organic solvents preferably used in the production method of the present disclosure include
aliphatic solvents, such as pentane, hexane, heptane, cyclohexane, and petroleum ether;
halogenated-aliphatic solvents, such as dichloromethane, dichloroethane, chloroform, fluorotrichloromethane, 1,1,2-trichlorotrifluoroethane, 2-chloro-1,2-dibromo-1,1,2-trifluoroethane, 1,2-dibromohexafluoropropane, 1,2-dibromotetrafluoroethane, 1,1-difluorotetrachloroethane, 1,2-difluorotetrachloroethane, heptafluoro-2,3,3-trichlorobutane, 1,1,1,3-tetrachlorotetrafluoropropane, 1,1,1-trichloropentafluoropropane, 1,1,1-trichlorotrifluoroethane, and polychlorotrifluoroethylene;
ester solvents, such as methyl formate, ethyl formate, methyl acetate, ethyl acetate, propyl acetate, methyl propionate, γ-butyrolactone, and propylene carbonate;
nitrile solvents, such as acetonitrile and propionitrile; aromatic solvents, such as benzene, chlorobenzene, toluene, dichlorobenzene, fluorobenzene, and nitrobenzene;
ether solvents, such as diethyl ether, dipropyl ether, tetrahydrofuran, cyclopentyl methyl ether (CPME), and methyl tertiary butyl ether (MTBE); and
acetone, N,N-dimethylformamide (DMF), dimethylsulfoxide (DMSO), nitromethane, N,N-diethylformamide, N,N-dimethylacetamide, 1-methyl-2-pyrrolidone, 1,3-dimethyl-2-imidazolidinone (DMI), tetramethyl urea, 1,3-dimethylpropylene urea, and hexamethylphosphoramide (HMPA).

These may be used alone or in any combination of two or more (e.g., as mixed solvents).

The reaction temperature of the fluorination step A is
typically lower than 120°C,
preferably lower than 100°C,
more preferably lower than 90°C, and
still more preferably lower than 80°C, from the standpoint of yield of the target fluorinated organic compound (1), as well as from economic and other standpoints.

The reaction temperature of the fluorination step A is
preferably -20°C or higher,
more preferably 0°C or higher, and
still more preferably 5°C or higher, from the standpoint of yield of the target fluorinated organic compound (1), as well as from economic and other standpoints.

The suitable reaction time of the fluorination step A can vary depending on the substrate, target product, and the like. From the standpoint of yield of the target fluorinated organic compound (1), as well as from economic and other standpoints, the suitable reaction time can be, for example,
typically less than 80 hours,
preferably less than 60 hours,
more preferably less than 40 hours,
still more preferably less than 30 hours, and
even more preferably less than 24 hours.

The suitable reaction time of the fluorination step A can vary depending on the substrate, target product, and the like. From the standpoint of yield of the target fluorinated organic compound (1), as well as from economic and other standpoints, the suitable reaction time can be, for example,
preferably 0.5 hours or more,
more preferably 1 hour or more, and
still more preferably 2 hours or more.

According to the reaction of the present disclosure, the amount of fluorine atom introduced per mole of the substrate may be preferably 0.65 mol or more, more preferably 0.70 mol or more, still more preferably 0.75 mol or more, and even more preferably 0.80 mol or more. The upper limit of the amount of fluorine atom introduced per mole of the substrate is not limited and can be typically 10 mol or less, although it depends on the number of reaction sites.

### Composition

The composition of the present disclosure comprises
(1) an organic solvent and
(2) IF₅ in an amount of 1.8 mol or more per liter of the organic solvent.

The composition of the present disclosure preferably comprises
(1) an organic solvent in an amount greater than 20 mass% based on the total amount of the composition taken as 100 mass%;
(2) IF₅ in an amount of 1.8 mol or more per liter of the organic solvent; and
(3) an acid or a base, or a combination thereof.

These compositions of the present disclosure are preferably liquid compositions without limitation, from the standpoint of yield of the target fluorinated organic compound (1), as well as from economic and other standpoints.

Below, the composition of the present disclosure is described.

The composition can be used in the production method of the present disclosure, the details of which can be understood based on common technical knowledge, also with reference to the above descriptions of the production method of the present disclosure, in addition to the following descriptions.

The organic solvent may be those mentioned above for the production method.

The organic solvent is preferably an aliphatic hydrocarbon, an aromatic hydrocarbon, a halogenated hydrocarbon, an ether, a ketone, an ester, a nitrile, or an amide, or a combination of two or more thereof, from the standpoint of yield of the target fluorinated organic compound (1), as well as from economic and other standpoints.

Examples of aliphatic hydrocarbons include pentane, hexane, heptane, cyclohexane, and petroleum ether.

Examples of aromatic hydrocarbons include benzene, chlorobenzene, toluene, dichlorobenzene, fluorobenzene, and nitrobenzene.

Examples of halogenated hydrocarbons include dichloromethane, dichloroethane, chloroform, fluorotrichloromethane, 1,1,2-trichlorotrifluoroethane, 2-chloro-1,2-dibromo-1,1,2-trifluoroethane, 1,2-dibromohexafluoropropane, 1,2-dibromotetrafluoroethane, 1,1-difluorotetrachloroethane, 1,2-difluorotetrachloroethane, heptafluoro-2,3,3-trichlorobutane, 1,1,1,3-tetrachlorotetrafluoropropane, 1,1,1-trichloropentafluoropropane, 1,1,1-trichlorotrifluoroethane, and polychlorotrifluoroethylene.

Examples of ethers include diethyl ether, dipropyl ether, tetrahydrofuran, cyclopentyl methyl ether (CPME), and methyl tertiary butyl ether (MTBE).

Examples of ketones include acetone.

Examples of esters include methyl formate, ethyl formate, methyl acetate, ethyl acetate, propyl acetate, methyl propionate, γ-butyrolactone, and propylene carbonate.

Examples of nitriles include acetonitrile and propionitrile.

Examples of amides include N,N-dimethylformamide (DMF), N,N-diethylformamide, N,N-dimethylacetamide, 1-methyl-2-pyrrolidone, 1,3-dimethyl-2-imidazolidinone (DMI), tetramethyl urea, and 1,3-dimethylpropylene urea.

The organic solvent is preferably an aliphatic hydrocarbon, an aromatic hydrocarbon, a halogenated hydrocarbon, or a nitrile, or a combination of two or more thereof, from the standpoint of yield of the target fluorinated organic compound (1), as well as from economic and other standpoints.

The lower limit of the amount of the organic solvent in the composition of the present disclosure is
typically greater than 20.0 mass%,
preferably 20.5 mass% or more,
more preferably 21.0 mass% or more,
still more preferably 22.5 mass% or more,
even more preferably 23.0 mass% or more,
particularly preferably 23.5 mass% or more,
more particularly preferably 24.0 mass% or more, and even more particularly preferably 24.5 mass% or more, from the standpoint of yield of the target fluorinated organic compound (1), as well as from economic and other standpoints.

The upper limit of the amount of the organic solvent in the composition of the present disclosure is not limited and is typically 80.0 mass% or less,
preferably 75.0 mass% or less,
more preferably 70.0 mass% or less,
still more preferably 65.0 mass% or less,
even more preferably 60.0 mass% or less,
particularly preferably 55.0 mass% or less,
more particularly preferably 50.0 mass% or less, and still more particularly preferably 45.0 mass% or less, from the standpoint of yield of the target fluorinated organic compound (1), as well as from economic and other standpoints.

The lower limit of the amount of IF₅ per liter of the organic solvent is
1.8 mol or more,
preferably 2.0 mol or more,
more preferably 2.2 mol or more,
still more preferably 2.5 mol or more,
even more preferably 2.7 mol or more,
particularly preferably 3.0 mol or more, and
more particularly preferably 3.5 mol or more, from the standpoint of yield of the target fluorinated organic compound (1), as well as from economic and other standpoints.

The upper limit of the amount of IF₅ per liter of the organic solvent is not limited, and is
typically 100 mol or less,
preferably 70 mol or less,
more preferably 50 mol or less,
still more preferably 30 mol or less,
even more preferably 20 mol or less,
particularly preferably 15 mol or less, and
more particularly preferably 10 mol/L or less, from the standpoint of yield of the target fluorinated organic compound (1), as well as from economic and other standpoints.

The concentration of IF₅ is not limited and is preferably approximately equal to the amount of the hydrogen atom-containing organic compound (2) (specifically, the molar ratio relative to the hydrogen atom-containing organic compound (2) is preferably in the range of 0.1 to 10.0, more preferably 0.3 to 5.0, and still more preferably 0.5 to 3.0), from the standpoint of yield of the target fluorinated organic compound (1), as well as from economic and other standpoints.

The acid is not limited and is preferably a Bronsted acid or a Lewis acid, or a combination thereof,
more preferably a Bronsted acid, and
still more preferably hydrogen fluoride, from the standpoint of yield of the target fluorinated organic compound (1), as well as from economic and other standpoints.

The Lewis acid can refer to one that is not an acid according to the Bronsted definition but is an acid according to the Lewis definition.

The base is not limited. From the standpoint of yield of the target fluorinated organic compound (1), as well as from economic and other standpoints, the base is
preferably an inorganic base or an organic base, or a combination thereof,
more preferably an organic base, and
still more preferably triethylamine or pyridine, or a combination thereof.

### Examples

The meanings of the symbols and abbreviations in the Examples are shown below.

### Et₃N: triethylamine.

### Examples 1 and 2 and Comparative Example A

After toluene (Examples 1 and 2: 0.87 g, 1.0 mL; Comparative Example A: 8.7 g, 10 mL) was placed in a reactor, O-n-decyl S-methyl dithiocarbonate (1.0 g, 4.03 mmol) was added thereto. Then, IF₅-Et₃N-3HF (1.54 g, 4.03 mmol) was added with stirring, and the mixture was reacted at room temperature for a predetermined time (see Table 1). After the reaction was ended with an aqueous potassium hydroxide solution, the yields of n-decyl trifluoromethyl ether (CF₃ form) and methyl n-decyloxydifluoromethyl sulfide (CF₂ form) were analyzed by F-NMR. In Examples 1 and 2, the content of the organic solvent in the composition used (the substrate, fluorinating agent, and organic solvent) was 25.5 mass%. In Comparative Example A, the content of the organic solvent in the composition used (the substrate, fluorinating agent, and organic solvent) was 77.4 mass%.

### Examples 3 and 4 and Comparative Example B

After dichloromethane (Examples 3 and 4: 0.66 g, 0.5 mL; Comparative Example B: 13.3 g, 10 mL) was placed in a reactor, N-butyl-N-propionyldithiocarbamic acid methyl ester (0.5 g, 2.28 mmol) was added thereto. Then, IF₅-Et₃N-3HF (0.87 g, 2.28 mmol) was added with stirring, and the mixture was reacted at room temperature for a predetermined time (see Table 1). After the reaction was ended with an aqueous potassium hydroxide solution, the yield of N-butyl-N-trifluoromethylpropanamide (CF₃ form) was analyzed by F-NMR. In Examples 3 and 4, the content of the organic solvent in the composition used (the substrate, fluorinating agent, and organic solvent) was 32.5 mass%. In Comparative Example B, the content of the organic solvent in the composition used (the substrate, fluorinating agent, and organic solvent) was 90.7 mass%.

### Example 5 and Comparative Example C

After toluene (Example 5: 0.26 g, 0.3 mL; Comparative Example C: 2.6 g, 3.0 mL) was placed in a reactor, 4-chloro-2,6-difluorothionobenzoic acid 3,4,5-trifluorophenyl ester (0.3 g, 0.89 mmol) was added thereto. Then, IF₅-Et₃N-3HF (340 mg, 0.89 mmol) was added with stirring, and the mixture was reacted at room temperature for a predetermined time (see Table 1). After the reaction was ended with an aqueous potassium hydroxide solution, the yield of 4-[difluoro(3,4,5-trifluorophenoxy)methyl]-1-chloro-3,5-difluorobenzene (CF₂ form) was analyzed by F-NMR. In Example 5, the content of the organic solvent in the composition used was 28.9 mass%. In Comparative Example C, the content of the organic solvent in the composition used was 80.3 mass%.

### Example 6 and Comparative Example D

After ethyl acetate (Example 6: 0.90 g, 1.0 mL; Comparative Example D: 2.6 g, 2.92 mL) was placed in a reactor, O-[4-(4-penthylcyclohexyl)cyclohexyl] methylsulfanylmethanethioate (1.0 g, 2.92 mmol) was added thereto. Then, IF₅-Et₃N-3HF (1.45 g, 3.79 mmol) was added with stirring, and the mixture was reacted at 40°C for a predetermined time (see Table 1). After the reaction was ended with an aqueous potassium hydroxide solution, the yield of 1-methyl-4-[4-(trifluoromethoxy)cyclohexyl]cyclohexane (CF₃ form) was analyzed by F-NMR. In Example 6, the content of the organic solvent in the composition used (the substrate, fluorinating agent, and organic solvent) was 26.9 mass%. In Comparative Example D, the content of the organic solvent in the composition used (the substrate, fluorinating agent, and organic solvent) was 51.5 mass%.

Table 1 shows the results of the Examples above.

**Table 1**

| Entry | Time (h) | Substrate concentration (mol/L) | IF₅ concentration (mol/L) | % Yield (F-NMR) | |
|---|---|---|---|---|---|
| | | | | CF₃ form | CF₂ form |
| Ex. 1 | 3.0 | 4.03 | 4.03 | 22 | 65 |
| Ex. 2 | 6.5 | 4.03 | 4.03 | 23 | 64 |
| Comp. Ex. A | 10.0 | 0.40 | 0.40 | 5 | 50 |
| Ex. 3 | 0.3 | 4.56 | 4.56 | 71 | 0 |
| Ex. 4 | 6.0 | 4.56 | 4.56 | 75 | 0 |
| Comp. Ex. B | 6.0 | 0.23 | 0.23 | 54 | 0 |
| Ex. 5 | 19.5 | 2.96 | 2.96 | 0 | 49 |
| Comp. Ex. C | 19.5 | 0.30 | 0.30 | 0 | 0 |
| Ex. 6 | 10.0 | 2.92 | 3.79 | 54 | 0 |
| Comp. Ex. C | 10.0 | 1.00 | 1.30 | 42 | 0 |

### Example 7

Acetonitrile (1.1 g, 1.4 mL) was placed in a reactor, and N-butyl-N-propionyldithiocarbamic acid methyl ester (1.4 g, 6.39 mmol) was added thereto. Then, IF₅-Et₃N-3HF (2.45 g, 6.39 mmol) was added with stirring, and the mixture was reacted at room temperature for a predetermined time (see Table 2). After the reaction was ended with an aqueous potassium hydroxide solution, the yield of N-butyl-N-trifluoromethylpropanamide (CF₃ form) was analyzed by F-NMR. In Example 7, the content of the organic solvent in the composition used (the substrate, fluorinating agent, and organic solvent) was 22.2 mass%.

### Example 8

After n-heptane (1.4 mL) was placed in a reactor, N-butyl-N-propionyldithiocarbamic acid methyl ester (1.4 g, 6.39 mmol) was added thereto. Then, IF₅-Et₃N-3HF (2.45 g, 6.39 mmol) was added with stirring, and the mixture was reacted at room temperature for a predetermined time (see Table 2). After the reaction was ended with an aqueous potassium hydroxide solution, the yield of N-butyl-N-trifluoromethylpropanamide (CF₃ form) was analyzed by F-NMR.

### Example 9

After 1,2-dimethoxyethane (0.43 g, 0.5 mL) and acetonitrile (0.39 g, 0.5 mL) were added to a reactor, 4-chloro-2,6-difluorothiobenzoic acid 3,4,5-trifluorophenyl ester (1.01 g, 3.0 mmol) was added thereto. Then, IF₅-Et₃N-3HF (1.73 g, 4.52 mmol) was added with stirring, and the mixture was reacted at 50°C for 16.0 hours (see Table 2). After the reaction was ended with an aqueous potassium hydroxide solution, the yield of 4-[difluoro(3,4,5-trifluorophenoxy)methyl]-1-chloro-3,5-difluorobenzene (CF₂ form) was analyzed by F-NMR. In Example 9, the content of the organic solvent in the composition used (the substrate, fluorinating agent, and organic solvent) was 23.0 mass%.

### Example 10 and Comparative Example E

After ethyl acetate (Example 10: 0.90 g, 1.0 mL; Comparative Example E: 2.69 g, 3.0 mL) was placed in a reactor, IF₅-Et₃N-3HF (1.75 g, 4.57 mmol) was added thereto. At this point, the content of the organic solvent in the composition used was 34.0 mass% in Example 10 and 60.6 mass% in Comparative Example E. Then, N-butyl-N-propionyldithiocarbamic acid methyl ester (1.0 g, 4.57 mmol) was added with stirring, and the mixture was reacted at 40°C for 16.0 hours (see Table 2). At this point, the content of the organic solvent in the composition used was 24.7 mass% in Example 10 and 49.5 mass% in Comparative Example E. After the reaction was ended with an aqueous potassium hydroxide solution, the yield of N-butyl-N-trifluoromethylpropanamide (CF₃ form) was analyzed by F-NMR.

### Example 11 and Comparative Example F

After toluene (Example 11: 0.26 g, 0.3 mL; Comparative Example F: 2.6 g, 3.0 mL) was placed in a reactor, IF₅-Et₃N-3HF (524 mg, 1.37 mmol) was added thereto. At this point, the content of the organic solvent in the composition used was 32.7 mass% in Example 11 and 83.0 mass% in Comparative Example F. Then, N-butyl-N-propionyldithiocarbamic acid methyl ester (0.3 g, 1.37 mmol) was added with stirring, and the mixture was reacted at room temperature for 16.0 hours (see Table 2). At this point, the content of the organic solvent in the composition used was 23.8 mass% in Example 11 and 75.7 mass% in Comparative Example F. After the reaction was ended with an aqueous potassium hydroxide solution, the yield of N-butyl-N-trifluoromethylpropanamide (CF₃ form) was analyzed by F-NMR. In Example 11, the content of the organic solvent in the composition used was 33.2 mass%. In Comparative Example F, the content of the organic solvent in the composition used was 83.2 mass%.

**Table 2**

| Entry | Time (h) | Substrate concentration (mol/L) | IF₅ concentration (mol/L) | % Yield (F-NMR) | |
|---|---|---|---|---|---|
| | | | | CF₃ form | CF₂ form |
| Ex. 7 | 16.0 | 4.56 | 4.56 | 52 | 0 |
| Ex. 8 | 16.0 | 4.56 | 4.56 | 59 | 0 |
| Ex. 9 | 16.0 | 3.00 | 4.52 | 0 | 93 |

**Table 3**

| Entry | Time (h) | Substrate concentration (mol/L) | IF₅ concentration (mol/L) | % Yield (F-NMR) | |
|---|---|---|---|---|---|
| | | | | CF₃ form | CF₂ form |
| Ex. 10 | 16.0 | 4.57 | 4.57 | 55 | 0 |
| Comp. Ex. E | 16.0 | 1.52 | 1.52 | 32 | 0 |
| Ex. 11 | 16.0 | 4.57 | 4.57 | 72 | 0 |
| Comp. Ex. F | 16.0 | 0.46 | 0.46 | 39 | 0 |

## Claims

1. A production method for a fluorinated organic compound (1), comprising
step A of fluorinating a hydrogen atom-containing organic compound (2) using IF₅ in a liquid composition comprising an organic solvent, the amount of the hydrogen atom-containing organic compound (2) being 1.8 mol or more per liter of the organic solvent.

2. The production method for a fluorinated organic compound according to claim 1, wherein the fluorination is performed using IF₅, an acid, and a base.

3. The production method for a fluorinated organic compound according to claim 1 or 2, wherein the fluorination is performed using IF₅, HF, and an organic base.

4. The production method for a fluorinated organic compound according to any one of claims 1 to 3, wherein the fluorination is performed using IF₅, HF, and an amine.

5. The production method according to any one of claims 1 to 4, wherein the hydrogen atom-containing organic compound (2) is a compound having a partial structure: -C(=S)-Y-,
wherein Y is O, S, or a single bond.

6. The production method according to any one of claims 1 to 5, wherein the fluorinated organic compound (1) is a compound having at least one partial structure: -CF₂- formed in step A.

7. The production method according to any one of claims 1 to 6, wherein the fluorinated organic compound (1) is a compound having at least one partial structure: -CF₃ formed in step A.

8. The production method according to any one of claims 1 to 7, wherein the concentration of the organic compound (2) is 2.5 mol or more per liter of the organic solvent.

9. The production method according to any one of claims 1 to 8, wherein the organic solvent is an aprotic solvent.

10. The production method according to any one of claims 1 to 9, wherein the reaction temperature of the fluorination step A is lower than 120°C.

11. A composition comprising
(1) an organic solvent and
(2) IF₅ in an amount of 1.8 mol or more per liter of the organic solvent.

12. A composition comprising:
(1) an organic solvent in an amount greater than 20.0 mass% based on the total amount of the composition taken as 100 mass%;
(2) IF₅ in an amount of 1.8 mol or more per liter of the organic solvent; and
(3) an acid or a base, or a combination thereof.

13. The composition according to claim 11 or 12, wherein the organic solvent is an aliphatic hydrocarbon, an aromatic hydrocarbon, a halogenated hydrocarbon, an ether, a ketone, an ester, a nitrile, or an amide, or a combination of two or more thereof.

14. The composition according to any one of claims 11 to 13, wherein the organic solvent is an aliphatic hydrocarbon, an aromatic hydrocarbon, a halogenated hydrocarbon, a nitrile, or a combination of two or more thereof.

15. The composition according to any one of claims 11 to 14, wherein the acid is a Bronsted acid or a Lewis acid, or a combination thereof.

16. The composition according to any one of claims 11 to 15, wherein the acid is a Bronsted acid.

17. The composition according to any one of claims 11 to 16, wherein the acid is hydrogen fluoride.

18. The composition according to any one of claims 11 to 17, wherein the base is an inorganic base or an organic base, or a combination thereof.

19. The composition according to any one of claims 11 to 18, wherein the base is an organic base.

20. The composition according to any one of claims 11 to 19, wherein the base is triethylamine or pyridine, or a combination thereof.

21. The composition according to any one of claims 11 to 20, which is a liquid.
